# FASCICULE DE BREVET EUROPEEN

(11) **EP 2 459 221 B1**
(45) Date de publication et mention de la délivrance du brevet: **22.01.2014**
(21) Numéro de dépôt: 10762950.3
(22) Date de dépôt: 29.07.2010
(51) Int. Cl.: A61K 47/10, A61K 31/519, A61K 9/00, A61K 47/14

(54) **FORMULATION PHARMACEUTIQUE**
PHARMAZEUTISCHE ZUSAMMENSETZUNG
PHARMACEUTICAL FORMULATION

(30) Priorité: 30.07.2009 FR 0903742
(43) Date de publication de la demande: 06.06.2012
(73) Titulaire: SANOFI, 75008 Paris (FR)
(72) Inventeur: BURNOUF, Jean-Pierre, F-75013 Paris (FR); BENARD, Tsiala, F-75013 Paris (FR)
(74) Mandataire: Jacobson, Claude
(86) Numéro de dépôt international: PCT/FR2010/051611
(87) Numéro de publication internationale: WO 2011/012816

(56) Documents cités:
- WO-A2-2008/102075

## Description

La présente demande est relative à une formulation pharmaceutique comprenant en tant que principe actif le composé de formule (I) : 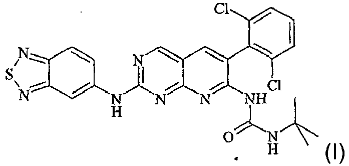 ou un sel de ce composé avec un acide pharmaceutiquement acceptable.

### [Problème technique]

L'administration d'un agent anticancéreux par voie intraveineuse est la voie d'administration préférée en oncologie car elle permet une diffusion rapide de l'agent dans le flux sanguin. Il s'agit parfois de la seule forme d'administration lorsque l'agent ne présente pas une biodisponibilité suffisante lorsqu'il est administré par une autre voie telle que la voie orale.

Le composé de formule (I) présente une faible solubilité dans différents milieux (voir **Tableau I**).

**Tableau I**

| milieu | solubilité [mg/g] |
|---|---|
| eau | <0,006 |
| PEG 400 | 9,5 |
| propylène glycol | 1 |
| éthanol | 3,8 |
| tert-butanol | 1,2 |

Or, il est nécessaire de pouvoir disposer d'une formulation pharmaceutique stable qui soit manipulable et qui permette d'administrer une quantité suffisante et efficace du composé de formule (I), de l'ordre de quelques centaines de mg. Il est également nécessaire que la formulation pharmaceutique soit stable chimiquement et physiquement, de même que la solution pour perfusion obtenue après dilution dans un milieu isotonique.

### [Etat de la technique]

WO 2007/003765 décrit des dérivés de pyrido[2,3-d]pyrimidine de formule (A) : 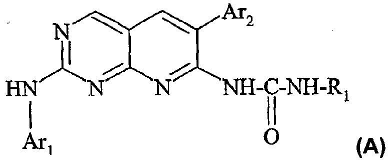 pouvant être administrés sous forme de solution.

WO 08102075 décrit l'utilisation du composé de formule (I) dans la traitement de leucémies. Le composé peut être administré sous forme de solution. Celle-ci peut être l'une des suivantes :
- PEG 400 22% / SOLUTOL^{®} HS15 5% / G5 73% pour le traitement par voie iv de souris portant des tumeurs Kasumi1, G5 étant une solution de glucose à 5% dans l'eau ;
- LABRASOL^{®} 21% / SOLUTOL^{®} HS15 5% / HCl 0,001 N 74% pour le traitement par voie orale de souris portant des tumeurs Kasumi1 ou KG1 ;
- DMSO 5% / TWEEN^{®} 80 10% / H₂O 85% pour le traitement par voie intra-péritonéale de souris portant des tumeurs EOL-1.

Les formulations décrites sont donc administrées telles quelles à des souris et ne sont pas destinées à être diluées pour former une solution pour perfusion.

### [Brève description de l'invention]

L'invention est relative à une formulation pharmaceutique comprenant le composé de formule (I) sous forme de base ou sous forme d'un sel d'un acide pharmaceutiquement acceptable solubilisé dans un mélange d'éthanol et d'un tensioactif comprenant un mélange du mono- et du di-ester polyéthoxylé de l'acide 12-hydroxystéarique décrits ci-après.

Le tensioactif comprend en poids de 35 à 55% de mono- et de diester et de 30 à 40% de polyéthylène glycol H(OCH₂CH₂)ₙ-OH. Il comprend en poids comme composants majoritaires de 35 à 55% de mono- et de diester et de 30 à 40% de polyéthylène glycol H(OCH₂CH₂)ₙ-OH, ainsi que d'autres composés formant le complément à 100%. Il comprend en poids comme de 10 à 20% de monoester, de 25 à 35% de diester et de 30 à 40% de polyéthylène glycol H(OCH₂CH₂)ₙ-OH ainsi que d'autres composés formant le complément à 100%.

Il peut s'agir du tensioactif macrogol 15 hydroxystéarate dans un rapport en poids tensioactif/éthanol allant de 25/75 à 80/20, de préférence de 73/27 à 77/23.

Le rapport tensioactif/éthanol va de 73/27 à 77/23 et la concentration en composé de formule (I) va de 5 à 25 mg/ml.

La formulation pharmaceutique est destinée à être diluée pour former une solution pour perfusion.

L'invention est aussi relative à un procédé de préparation de la formulation pharmaceutique comprenant les étapes suivantes :
- le tensioactif est chauffé jusqu'à devenir liquide ;
- l'éthanol est ajouté ;
- le mélange tensioactif/éthanol est refroidi jusqu'à la température ambiante ;
- le composé de formule (I) est ajouté au mélange refroidi ;
- le mélange final est stérilisé, de préférence par filtration.

L'invention est aussi relative à une solution pour perfusion comprenant le composé de formule (I) sous forme de base ou sous forme d'un sel d'un acide pharmaceutiquement acceptable obtenue par dilution de 1 volume de la solution pharmaceutique dans 20 à 500 volumes d'une solution isotonique: Le composé de formule (I) à une concentration allant de 0,01 à 1,2 mg/ml, le tensioactif tel à une concentration allant de 0,48 à 37 mg/ml et l'éthanol à une concentration allant de 0,35 à 35 mg/ml sont dilués dans la solution isotonique. La solution pour perfusion est destinée à être administrée à un être humain.

L'invention est aussi relative au procédé de préparation de la solution pour perfusion consistant à diluer 1 volume de la solution pharmaceutique dans 20 à 500 volumes de la solution isotonique

L'invention est aussi relative à un flacon contenant la solution pharmaceutique et à une poche de perfusion contenant la solution pour perfusion.

L'invention est aussi relative à l'utilisation d'un tensioactif tel que défini précédemment pour la préparation d'une formulation pharmaceutique comprenant le composé de formule (I) sous forme de base ou sous forme d'un sel d'un acide pharmaceutiquement acceptable, cette formulation étant destinée à être diluée pour former une solution pour perfusion.

### [Description détaillée]

L'invention est relative à une formulation pharmaceutique comprenant le composé de formule (I) : 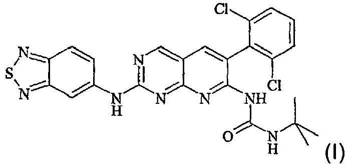 sous forme de base ou sous forme d'un sel d'un acide pharmaceutiquement acceptable, solubilisé dans un mélange d'éthanol et du tensioactif macrogol 15 hydroxystéarate dans un rapport en poids tensioactif/éthanol allant de 25/75 à 80/20, de préférence de 73/27 à 77/23.

La formulation pharmaceutique comprend le composé de formule (I) solubilisé dans un mélange :
- d'éthanol et
- d'un tensioactif comprenant un mélange du mono- et du di-ester polyéthoxylé de l'acide 12-hydroxystéarique ayant pour formules respectives : 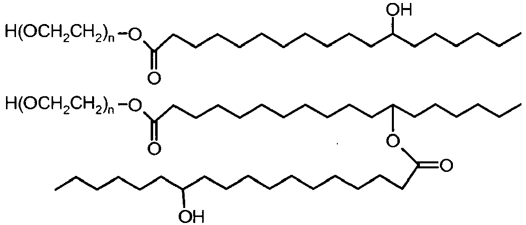
n étant un nombre entier allant de 15 à 16,
dans un rapport en poids tensioactif/éthanol allant de 25/75 à 80/20, de préférence de 73/27 à 77/23.

Le composé de formule (I) : 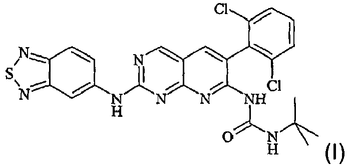 est un agent anticancéreux utilisable dans le traitement de leucémies décrit dans EP 1902054 B1**.** Il peut être sous forme de base (voir formule (I)) ou sous forme d'un sel d'un acide pharmaceutiquement acceptable ; voir à ce propos, « Remington's pharmaceutical sciences », 17ème éd., Mack Publishing Company, Easton, PA, 1985 et Berge et al., « Pharmaceutical salts » J.Pharm.Sci. 1977, 66, 1-19.

Le tensioactif hydrophile non ionique qui est utilisé est obtenu en faisant réagir vers 110-165°C l'acide 12-hydroxystéarique et l'oxyde d'éthylène en présence d'un catalyseur basique tel que K₂CO₃ selon l'enseignement de « Synthetic Detergents from Animal Fats. VIII. The Ethenoxylation of Fatty Acids and Alcohols » A.N.Wrigley J.Am.Oil.Chem.Soc. 1957, 34, 39-43 ou de J.V.Karabinos J.Am.Oil Chem.Soc. 1954, 31, 20-23. L'acide 12-hydroxystéarique est issu de l'hydrogénation de l'huile de ricin (« castor oil »). Voir aussi EP 0017059**.**

Le tensioactif contient majoritairement un mono ester de formule : 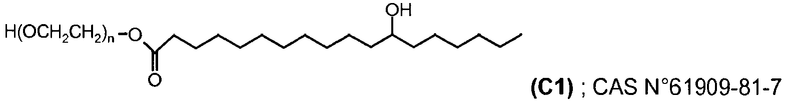 et un diester de formule :

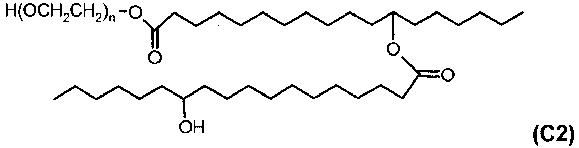

Par ailleurs, le tensioactif peut contenir aussi du polyéthylène glycol libre (C3 ; H(OCH₂CH₂)₁₅₋₁₆-OH). Le tensioactif peut ainsi comprendre en poids de 30 à 40% du polyéthylène glycol et de 60 à 70% de mono- et de diester.

Le tensioactif peut comprendre aussi d'autres composés issus de la réaction d'éthoxylation, notamment ceux de formules :

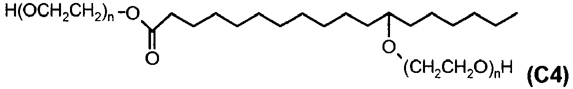

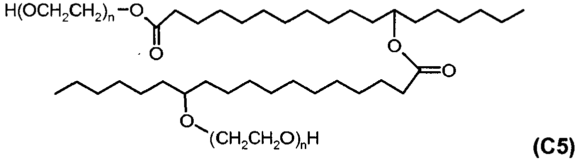

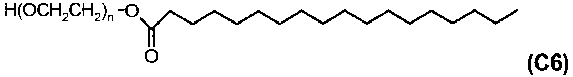

A titre d'exemples, les compositions de deux tensioactifs utilisables sont données dans le **Tableau II.** Le tensioactif comprend donc en poids comme composants majoritaires de 35 à 55% de mono- et de diester de l'acide 12-hydroxystéarique et de 30 à 40% de polyéthylène glycol H(OCH₂CH₂)ₙ-OH, ainsi que d'autres composés formant le complément à 100%.

**Tableau II**

| **Composés [%poids]** | **Exemple 1 de tensioactif** | **Exemple 2 de tensioactif** | **Fourchettes** |
|---|---|---|---|
| **C1 (monoester)** | 19 | 12 | 10-20 |
| **C2 (diester)** | 30 | 34 | 25-35 |
| **C3 (PEG)** | 35 | 35 | 30-40 |
| **C4** | 9 | 6 | complément à 100%* |
| **C5** | 3 | 6 | |
| **C6** | 3 | 2 | |
| **autres composés** | 1 | 5 | |

| | | | |
|---|---|---|---|
| * pour l'ensemble des composés C4-C6 + autres composés | | | |

Un exemple de tensioactif utilisable est le SOLUTOL^{®} HS15 commercialisé par la société BASF qui est décrit dans J.Pharm.Sci. 1998, 87(2), 200-208, Pharm.Res. 2004, 21(2), 201-230 (page 222), Int. J. Cancer 1995, 62, 436-442 ainsi que dans Cancer Res. 1991, 51, 897-902 et dont on trouvera en **annexe 1** la brochure technique. La pharmacopée européenne (PhEur 6.0) le décrit comme le macrogol 15 hydroxystéarate ; il est décrit comme un mélange de monoester et de diester de l'acide 12-hydroxystéarique et de macrogol obtenu par éthoxylation de l'acide 12-hydroxystéarique. Le nombre de moles d'oxyde d'éthylène ayant réagi avec l'acide est de 15 (valeur nominale). Il contient environ 30% poids de macrogol libre. Il se présente sous forme d'une pâte blanchâtre à la température ambiante qui devient liquide vers 30°C. La balance hydrophile-lipophile est d'environ 14-16. La concentration micellaire critique (CMC) est comprise entre 0,005 et 0,12%. Autres données : température de fusion : 25-30°C ; valeur de saponification : 56-63 ; teneur en hydroxyle : 90-110 ; indice d'iode : 2 ; viscosité à 30%poids dans l'eau à 25°C : 12 mPas environ.

Selon une forme de l'invention, la formulation pharmaceutique peut comprendre au moins un autre additif usuellement utilisé dans les formulations pharmaceutiques liquides. Il peut s'agir par exemple d'un antioxydant, d'un conservateur, d'un tampon.... Selon une autre forme de l'invention, la formulation pharmaceutique ne comprend que le tensioactif, l'éthanol et le composé de formule (I).

On peut préparer la formulation pharmaceutique de la façon suivante :
- le tensioactif est chauffé jusqu'à devenir liquide ;

La température à laquelle le tensioactif devient liquide varie en fonction du tensioactif et des proportions de mono- et de diester et le cas échéant du polyéthylène glycol libre. La température est comprise généralement entre 35 à 50°C (bornes incluses). - l'éthanol est ajouté ;

La quantité ajoutée est telle que le rapport tensioactif/éthanol soit celui donné plus haut.
- le mélange tensioactif/éthanol est refroidi jusqu'à la température ambiante;
- le composé de formule (I) est ajouté au mélange refroidi ;
- le mélange final est stérilisé. On peut utiliser avantageusement la stérilisation par filtration : voir à ce propos, « Pharmaceutical process validation », R.A.Nash, 3ème édition, Marcel Dekker Inc, isbn=0824708385, page 119 ou « Validation of pharmaceutical processes », J.P.Agalloco, 3ème édition, 2007, isbn=9780849370557, pages 151-152. La stérilisation par filtration ne dégrade pas le composé de formule (I) qui est sensible à la chaleur à l'inverse de la stérilisation par chauffage. Par exemple, dans le cas de la formulation avec un rapport 75/25, on a pu utiliser une filtration sur filtre de 0,22 µm.

La formulation pharmaceutique décrite plus haut est un concentrat destiné à être dilué pour former une solution pour perfusion. Elle peut être contenue dans un flacon en verre. La solution pour perfusion est préparée extemporanément en diluant le concentrat dans une solution isotonique adaptée à la perfusion (par exemple glucosée ou salée). La solution pour perfusion est généralement préparée sous forme d'une poche de perfusion par le personnel hospitalier quelques temps avant l'administration. La solution pour perfusion est une solution micellaire sursaturée en composé de formule (I) obtenue par dilution de 1 volume de concentrat dans 20 à 500 volumes de solution isotonique. Elle est utilisable dans le traitement des cancers humains.

Le tensioactif a pour fonction de solubiliser le composé de formule (I) dans la formulation et de stabiliser la solution pour perfusion (micellisation). La solubilité du composé de formule (I) augmente donc avec le rapport tensioactif/éthanol. Cependant, au-delà du rapport 80/20, la viscosité de la formulation augmente au point de rendre un prélèvement à la seringue plus difficile, voire impossible.

L'éthanol sert de cosolvant et a pour fonction de diminuer la viscosité du tensioactif ce qui améliore sa manipulabilité. En-deçà d'un rapport tensioactif/éthanol de 25/75, la quantité d'éthanol administré devient importante et la solubilité du composé de formule (I) devient trop faible. L'éthanol ne peut être remplacé par le PEG 300 ou 400 car le tensioactif et le PEG 300/400 ne sont pas miscibles aux rapports tensioactif/PEG allant de 25/75 à 50/50 ou bien alors le mélange tensioactif/PEG 300/400 est solide à température ambiante aux rapports allant de 60/40 à 75/25. De même, la dilution du composé de formule (I) dans le PEG seul ne permet pas de dépasser une solubilité supérieure à 7 mg/ml et la stabilité physique de la solution pour perfusion n'est pas satisfaisante (<24 h). Enfin, les études de stabilité chimique ont permis de montrer que le composé de formule (I) se dégrade moins rapidement, surtout en conditions accélérées à 25°C/60%HR (humidité relative) et 30°C/65%HR, dans la formulation selon l'invention qu'avec une formulation à base de PS80 ou de PS80/éthanol.

La formulation selon l'invention offre donc les avantages suivants :
- il s'agit d'une solution homogène ;
- elle permet d'atteindre une solubilité suffisante en composé de formule (I) pour pouvoir en administrer à un patient une quantité de l'ordre de quelques centaines de mg ;
- elle est manipulable et permet notamment un prélèvement à la seringue ;
- le composé de formule (I) ne se dégrade pas autant qu'avec d'autres tensioactifs (cf. **Tableau IV**) ;
- la solution pour perfusion obtenue à partir de la formulation est stable physiquement pendant une durée d'au moins 24 h à la température ambiante c'est-à-dire qu'elle ne présente aucun critère visible de précipitation ;
- la formulation peut être stérilisable sur filtre.

La concentration en composé de formule (I) dans la formulation pharmaceutique peut varier de 5 à 25 mg/ml. La solubilité dépend en fait du rapport tensioactif/éthanol. Des exemples de formulations pharmaceutiques selon l'invention sont les suivantes :
(A) rapport tensioactif / éthanol entre 73/27 à 77/23, par exemple de 75/25 ; composé de formule (I) : 5-25 mg/ml ;
(B) rapport tensioactif / éthanol : 50/50 ; composé de formule (I) : 5-10 mg/ml ;
(C) rapport tensioactif / éthanol : 25/75 ; composé de formule (I) : 5 mg/ml

La solution pour perfusion comprend le composé de formule (I) à une concentration allant de 0,01 à 1,2 mg/ml, le tensioactif à une concentration allant de 0,48 à 37 mg/ml et l'éthanol à une concentration allant de 0,35 à 35 mg/ml, dilués dans une solution isotonique. De préférence, elle comprend le composé de formule (I) à une concentration allant de 0,01 à 1,2 mg/ml, le tensioactif à une concentration allant de 1,4 à 35 mg/ml et l'éthanol à une concentration allant de 0,4 à 13 mg/ml, dilués dans une solution isotonique.

### [Figures]

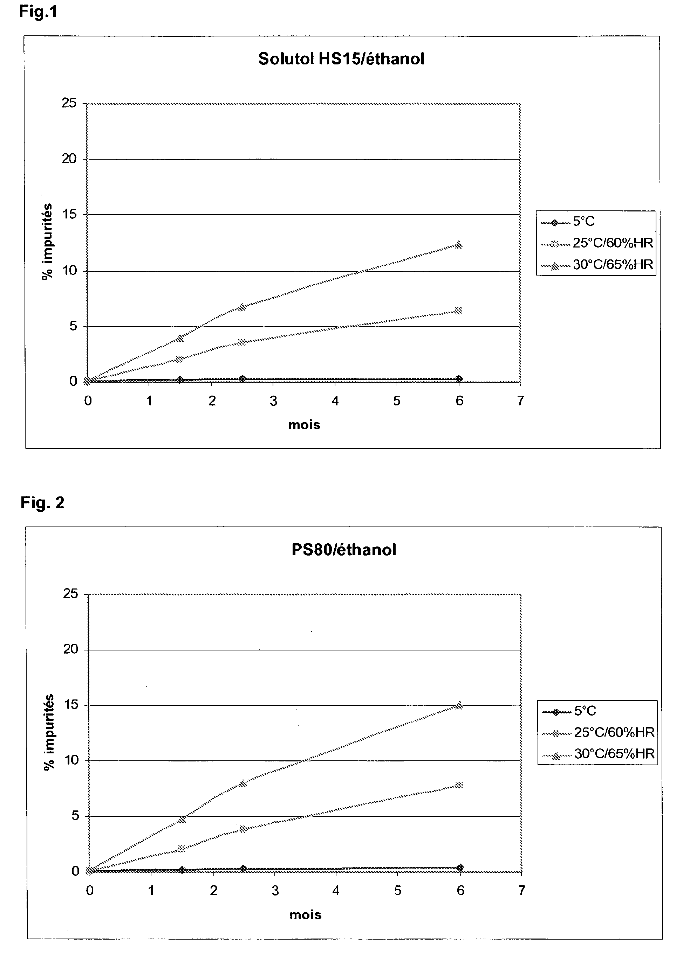

**Fig.1** **:** courbe d'évolution au cours du temps de la concentration en impuretés pour la formulation SOLUTOL^{®} HS15/éthanol ;
**Fig.2** **:** courbe d'évolution au cours du temps de la concentration en impuretés pour la formulation PS80/éthanol ;

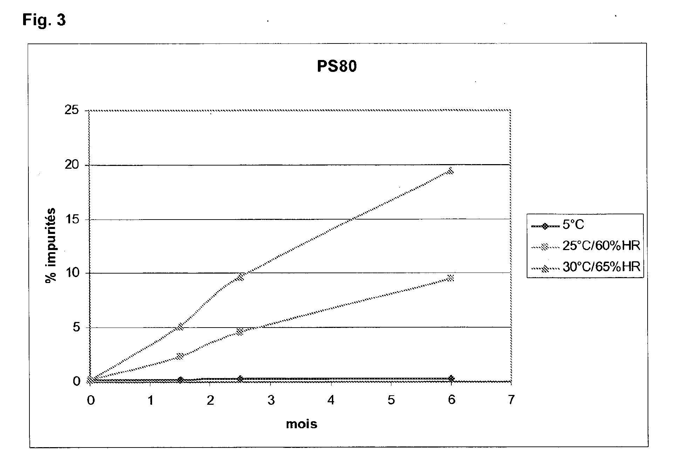

**Fig.3** **:** courbe d'évolution au cours du temps de la concentration en impuretés pour la formulation PS80.

### [Exemples]

### Préparation d'une formulation selon l'invention

Dans un réacteur en verre, on fait fondre le SOLUTOL^{®} HS15 à 40°C pendant environ 3 h, puis on arrête le chauffage et on inerte la cuve. On fait descendre la température de la cuve à 20°C, et, sans attendre le retour à 20°C, on ajoute l'éthanol au SOLUTOL^{®} HS15. On homogénéise ensuite pendant 30 min. On ajoute le composé de formule (I) (forme base) et on le dissout dans le mélange SOLUTOL^{®} HS15/éthanol et on laisse sous agitation pendant 3 h à température ambiante. On filtre ensuite sur filtre PVDF 0,22 µm et on stocke la solution pendant 24 h. On fait subir ensuite à la solution une filtration stérilisante sur filtre PVDF 0,22 µm.

### Autres formulations

Plusieurs formulations du composé de formule (I) sont comparées afin de déterminer celle qui permet d'atteindre la solubilité cible de 20 mg/g (**Tableau III**).

**Tableau III**

| **Formulation à base de** | **solubilité [mg/g]** | **commentaires** |
|---|---|---|
| PEG 400 | 9,5 | < cible |
| propylène glycol | 1 | < cible |
| éthanol | 3,8 | < cible |
| tert-butanol | 1,2 | < cible |
| micelles mixtes (Lécithine/Taurocholate Na 1/1) 0,1 M | 0,004 | < cible |
| albumine 4% | 0,013 | < cible |
| endolipid 20% (émulsion) | 0,26 | < cible |
| medialipid 20% (émulsion) | 0,13 | < cible |
| HydroxyPropylBetaCyclodextrine 40% | 0,087 | < cible |
| Polysorbate 80 grade pH 6,0 (PS80_{pH6}) | 29 | |
| Polysorbate 80 grade pH 3,5 (PS80_{pH3.5}) | 32 | |
| PS80_{pH6}/éthanol 75/25 (lot RSN2 : pureté = 99,7%) | 27,5 | |
| PS80_{pH6}/éthanol 75/25 (lot CER : pureté = 95,6%) | 30,3 | |
| PS80_{pH6}/éthanol 50/50 | 21,2 | |
| SOLUTOL^{®} HS15/éthanol 50/50 | 26,9 | |
| SOLUTOL^{®} HS15/éthanol 75/25 (lot RSN2 : pureté=99,7%) | 30,7 | |
| SOLUTOL^{®} HS15/éthanol 75/25 (lot CER : pureté=95,6%) | 33,8 | |
| SOLUTOL^{®} HS15/PEG400 20/80 | 14,4 | < cible |

Une étude de stabilité chimique par mesure de la teneur en impuretés par chromatographie liquide haute performance (HPLC) a été conduite sur les formulations permettant d'atteindre la solubilité cible. Pour ce faire, un dosage au cours du temps des impuretés présentes dans les trois formulations suivantes a été réalisé :
- SOLUTOL^{®} HS15/éthanol 50/50
- PS80_{pH6}/éthanol 50/50
- PS80

Les résultats sont rassemblés dans le **Tableau IV.**

**Tableau IV**

| **conditions** | **mois** | **SOLUTOL^{®} HS15/éthanol** | **PS80/éthanol** | **PS80** |
|---|---|---|---|---|
| **5°C** | 0 | 0,13 | 0,13 | 0,15 |
| | 1,5 | 0,14 | 0,15 | 0,19 |
| | 2,5 | 0,26 | 0,29 | 0,25 |
| | **6** | **0,32** | **0,37** | **0,31** |
| **25°C/60%HR** | 0 | 0,13 | 0,13 | 0,15 |
| | 1,5 | 2 | 2 | 2,3 |
| | 2,5 | 3,5 | 3,8 | 4,6 |
| | **6** | **6,4** | **7,8** | **9,5** |
| **30°C/65%HR** | 0 | 0,13 | 0,13 | 0,15 |
| | 1,5 | 4 | 4,7 | 5,1 |
| | 2,5 | 6,7 | 8 | 9,7 |
| | **6** | **12,4** | **15** | **19,4** |

On constate que la formulation associant le SOLUTOL^{®} HS15 et l'éthanol est la plus stable des trois.

### Stabilité physique et chimique de la dilution dans la poche de perfusion

Le concentrat SOLUTOL^{®} HS15/éthanol 75/25 (poids/poids) est dilué extemporanément dans la poche de perfusion. La stabilité physique et chimique de la dilution dans la poche de perfusion a été étudiée. Différents paramètres ont été évalués :
- la dilution : 0,04 mg/mL et 1 mg/mL
- le milieu de dilution (NaCl 0.9 % ou Glucose5 %)
- la température de stockage (5°C et 30°C)
- la durée de stockage

Quelles que soient les conditions testées, il a été montré que les poches sont stables chimiquement et physiquement au moins 72 h.

### ANNEXE 1

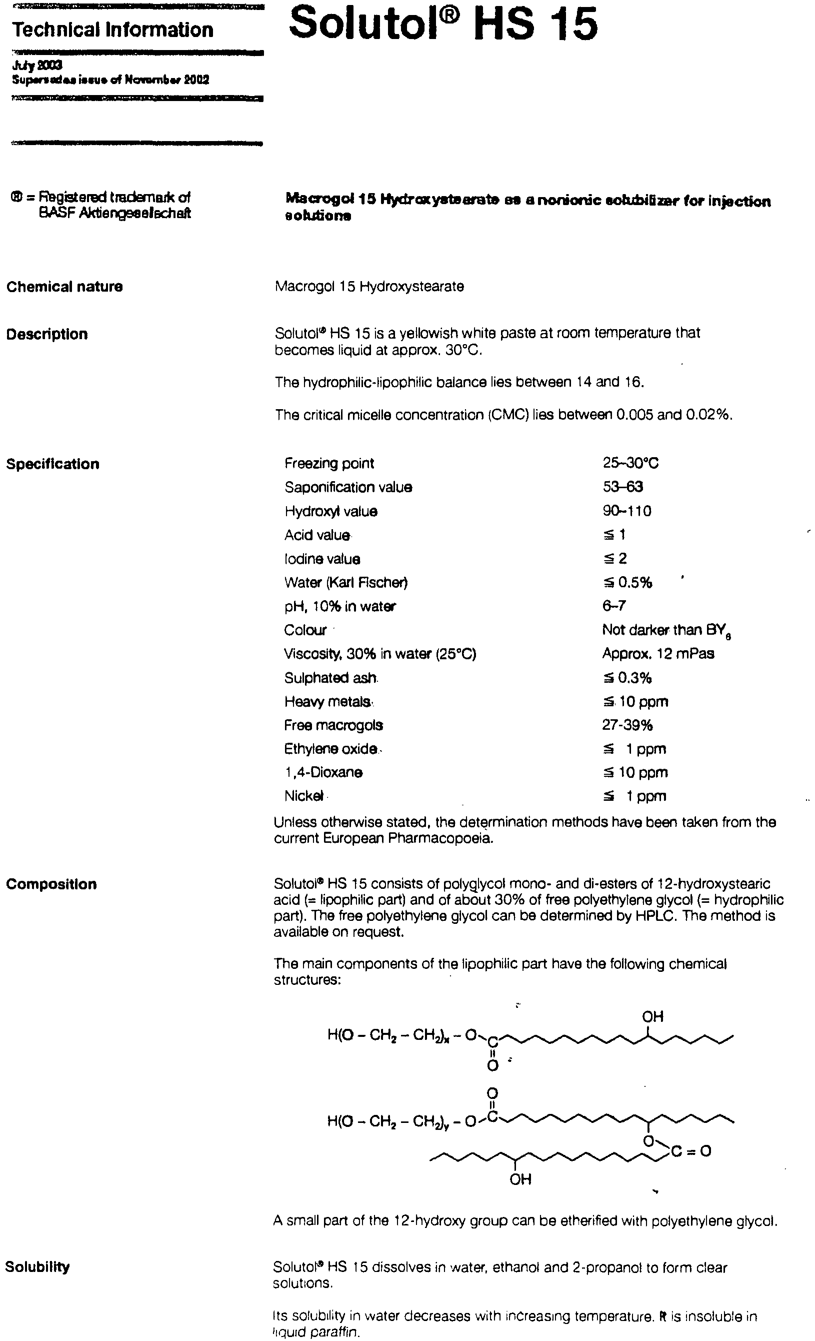

## Revendications

1. Formulation pharmaceutique comprenant le composé de formule (I) : 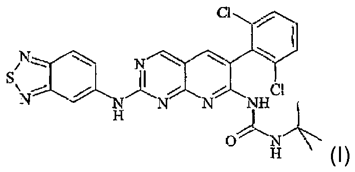 sous forme de base ou sous forme d'un sel d'un acide pharmaceutiquement acceptable,
solubilisé dans un mélange :
• d'éthanol et
• d'un tensioactif comprenant un mélange du mono- et du di-ester polyéthoxylé de l'acide 12-hydroxystéarique ayant pour formules respectives : 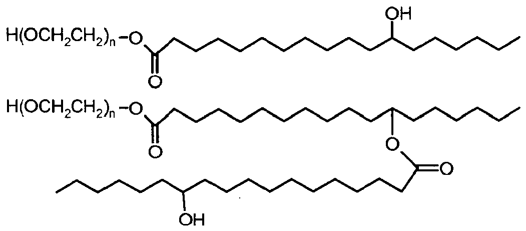
n étant un nombre entier allant de 15 à 16,
dans un rapport en poids tensioactif/éthanol allant de 25/75 à 80/20.

2. Formulation pharmaceutique selon la revendication 1, **caractérisée en ce que** le tensioactif est le tensioactif macrogol 15 hydroxystéarate.

3. Formulation pharmaceutique selon la revendication 1 ou 2, **caractérisée en ce que** le rapport en poids tensioactif/éthanol va de 73/27 à 77/23.

4. Formulation selon la revendication 2 dans laquelle le tensioactif comprend en poids de 35 à 55% de mono- et de diester et de 30 à 40% de polyéthylène glycol H(OCH₂CH₂)ₙ-OH.

5. Formulation selon la revendication 4 dans laquelle le tensioactif comprend en poids comme composants majoritaires de 35 à 55% de mono- et de diester et de 30 à 40% de polyéthylène glycol H(OCH₂CH₂)ₙ-OH, ainsi que d'autres composés formant le complément à 100%.

6. Formulation selon la revendication 1 ou 2 dans laquelle le tensioactif comprend en poids de 10 à 20% de monoester, de 25 à 35% de diester et de 30 à 40% de polyéthylène glycol H(OCH₂CH₂)ₙ-OH ainsi que d'autres composés formant le complément à 100%.

7. Formulation pharmaceutique selon la revendication 1 à 6 dans laquelle le rapport tensioactif/éthanol va de 73/27 à 77/23 et la concentration en composé de formule (I) va de 5 à 25 mg/ml.

8. Formulation pharmaceutique selon l'une des revendications 1 à 7 destinée à être diluée pour former une solution pour perfusion.

9. Procédé de préparation d'une formulation pharmaceutique selon l'une quelconque des revendications 1 à 8 comprenant les étapes suivantes :
- le tensioactif est chauffé jusqu'à devenir liquide ;
- l'éthanol est ajouté ;
- le mélange tensioactif/éthanol est refroidi jusqu'à la température ambiante ;
- le composé de formule (I) est ajouté au mélange refroidi ;
- le mélange final est stérilisé.

10. Procédé selon la revendication 9 dans lequel le mélange est stérilisé par filtration.

11. Solution pour perfusion comprenant le composé de formule (I) : 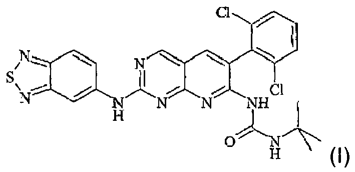 sous forme de base ou sous forme d'un sel d'un acide pharmaceutiquement acceptable,
obtenue par dilution de 1 volume de la solution pharmaceutique selon la revendication 1 à 8 dans 20 à 500 volumes d'une solution isotonique.

12. Solution pour perfusion comprenant le composé de formule (I) : 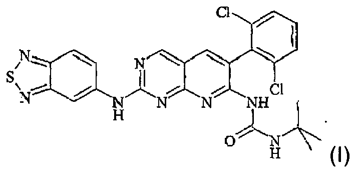 sous forme de base ou sous forme d'un sel d'un acide pharmaceutiquement acceptable, à une concentration allant de 0,01 à 1,2 mg/ml, le tensioactif tel que défini à l'une des revendications 1 à 7 à une concentration allant de 0,48 à 37 mg/ml et l'éthanol à une concentration allant de 0,35 à 35 mg/ml, dilués dans une solution isotonique.

13. Solution pour perfusion selon la revendication 11 ou 12 destinée à être administrée à un être humain.

14. Procédé de préparation d'une solution pour perfusion consistant à diluer 1 volume de la solution pharmaceutique selon la revendication 1 à 8 dans 20 à 500 volumes d'une solution isotonique

15. Flacon contenant la solution pharmaceutique telle que décrite à l'une des revendications 1 à 8.

16. Poche de perfusion contenant la solution pour perfusion de la revendication 11 à 13.

## Patentansprüche

1. Pharmazeutische Zusammensetzung, umfassend die Verbindung der Formel (I): 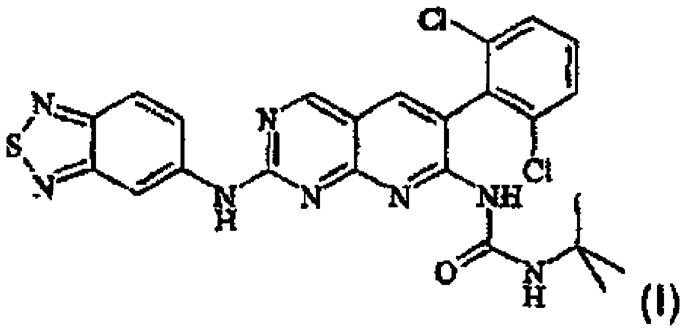 in Form einer Base oder in Form eines Salzes einer pharmazeutisch akzeptablen Säure,
gelöst in einer Mischung aus:
• Ethanol und
• einem oberflächenaktiven Mittel, das eine Mischung des polyethoxylierten Mono- und des Di-Esters der 12-Hydroxystearinsäure enthält, die als jeweilige Formel haben: 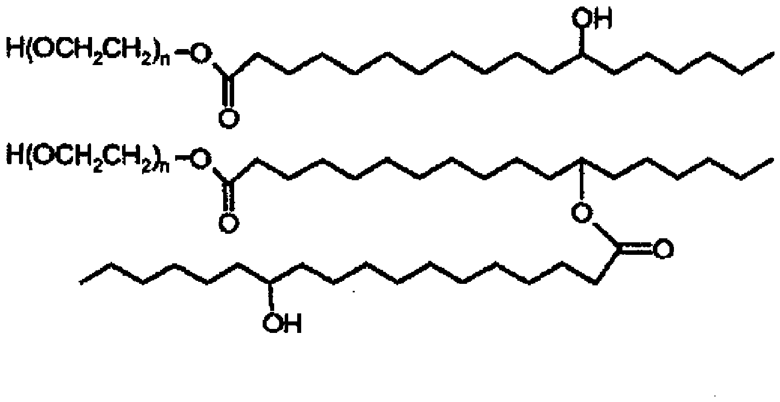
wobei n eine ganze Zahl von 15 bis 16 ist, bei einem Gewichtsverhältnis oberflächenaktives Mittel/Ethanol von 25/75 bis 80/20.

2. Pharmazeutische Zusammensetzung nach Anspruch 1, **dadurch gekennzeichnet, dass** das oberflächenaktive Mittel das oberflächenaktive Mittel Macrogol 15 hydroxystearat ist.

3. Pharmazeutische Zusammensetzung nach Anspruch 1 oder 2, **dadurch gekennzeichnet, dass** das Gewichtsverhältnis oberflächenaktives Mittel/Ethanol von 73/27 bis 77/23 geht.

4. Zusammensetzung nach Anspruch 2, bei der das oberflächenaktive Mittel bezogen auf das Gewicht 35 bis 55 % Mono- und Diester und 30 bis 40 % Polyethylenglykol H(OCH₂CH₂)ₙ-OH enthält.

5. Zusammensetzung nach Anspruch 4, bei der das oberflächenaktive Mittel bezogen auf das Gewicht als Hauptbestandteile 35 bis 55 % Mono- und Diester und 30 bis 40 % Polyethylenglykol H(OCH₂CH₂)ₙ-OH sowie andere Verbindungen, die das Komplement zu 100% bilden, umfasst.

6. Zusammensetzung nach Anspruch 1 oder 2, bei der das oberflächenaktive Mittel bezogen auf das Gewicht 10 bis 20 % Monoester, 25 bis 35 % Diester und 30 bis 40 % Polyethylenglykol H(OCH₂CH₂)ₙ-OH sowie andere Verbindungen, die das Komplement zu 100% bilden, umfasst.

7. Pharmazeutische Zusammensetzung nach einem der Ansprüche 1 bis 6, bei der das Verhältnis oberflächenaktives Mittel/Ethanol von 73/27 bis 77/23 geht und die Konzentration der Verbindung der Formel (I) von 5 bis 25 mg/ml geht.

8. Pharmazeutische Zusammensetzung nach einem der Ansprüche 1 bis 7, die vorgesehen ist, aufgelöst zu werden, um eine Infusionslösung zu bilden.

9. Verfahren zur Herstellung einer pharmazeutischen Zusammensetzung nach einem beliebigen der Ansprüche 1 bis 8, die folgenden Schritte umfassend:
- das oberflächenaktive Mittel wird erhitzt bis es flüssig wird;
- das Ethanol wird zugefügt;
- das Gemisch oberflächenaktives Mittel/Ethanol wird bis auf Umgebungstemperatur heruntergekühlt;
- das Endgemisch wird sterilisiert.

10. Verfahren nach Anspruch 9, bei dem das Gemisch durch Filtration sterilisiert wird.

11. Infusionslösung, die Verbindung der Formel (I) umfassend: 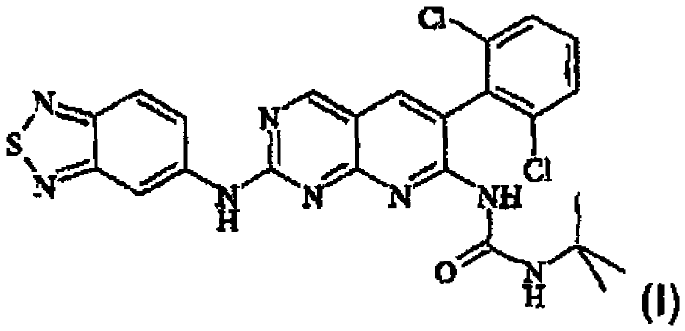 in Form einer Base oder in Form eines pharmazeutisch akzeptablen Salzes, erhalten durch Verdünnen von 1 Volumen der pharmazeutischen Lösung nach Anspruch 1 bis 8 in 20 bis 500 Volumen einer isotonischen Lösung.

12. Infusionslösung, die Verbindung der Formel (I) umfassend: 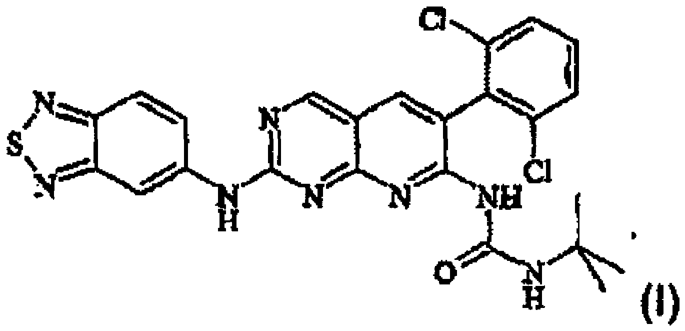 in Form einer Base oder in Form eines Salzes einer pharmazeutisch akzeptablen Säure, bei einer Konzentration, die von 0,01 bis 1,2 mg/ml geht, wobei das oberflächenaktive Mittel, wie es in einem der Ansprüche 1 bis 7 definiert ist, bei einer Konzentration von 0,48 bis 37 mg/ml und das Ethanol bei einer Konzentration von 0,35 bis 35 mg/ml in einer isotonischen Lösung verdünnt werden.

13. Infusionslösung nach Anspruch 11 oder 12, die vorgesehen ist, einem menschlichen Wesen verabreicht zu werden.

14. Verfahren zur Herstellung einer Infusionslösung, das darin besteht, 1 Volumen der pharmazeutischen Lösung nach Anspruch 1 bis 8 in 20 bis 500 Volumen einer isotonischen Lösung zu verdünnen.

15. Behälter, enthaltend eine pharmazeutische Lösung, wie sie in einem der Ansprüche 1 bis 8 beschrieben ist.

16. Infusionsbeutel, enthaltend eine Infusionslösung nach einem der Ansprüche 11 bis 13.

## Claims

1. Pharmaceutical formulation comprising the compound of the formula (I): 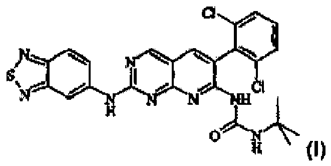 in the form of the base or in the form of a salt of a pharmaceutically acceptable acid,
solubilized in a mixture:
• of ethanol and
• of a surfactant comprising a mixture of the polyethoxylated mono- and diester of 12-hydroxystearic acid having the respective formulae: 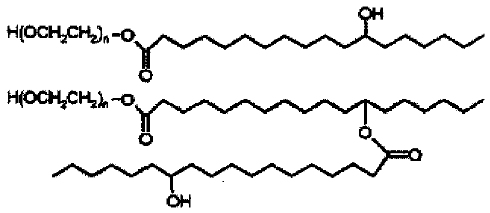
n being an integer in the range of from 15 to 16,
in a surfactant/ethanol weight ratio in the range of from 25/75 to 80/20.

2. Pharmaceutical formulation according to claim 1, **characterized in that** the surfactant is the surfactant macrogol 15-hydroxystearate.

3. Pharmaceutical formulation according to claim 1 or 2, **characterized in that** the surfactant/ethanol weight ratio is in the range of from 73/27 to 77/23.

4. Formulation according to claim 2, in which the surfactant comprises from 35 to 55 % by weight of mono- and diester and from 30 to 40 % by weight of polyethylene glycol H (OCH₂CH₂) ₙ-OH.

5. Formulation according to claim 4, in which the surfactant comprises as the main components from 35 to 55 % by weight of mono- and diester and from 30 to 40 % by weight of polyethylene glycol H(OCH₂CH₂)ₙ-OH, as well as other compounds to make up to 100 %.

6. Formulation according to claim 1 or 2, in which the surfactant comprises from 10 to 20 % by weight of monoester, from 25 to 35 % by weight of diester and from 30 to 40 % by weight of polyethylene glycol H(OCH₂CH₂)ₙ-OH, as well as other compounds to make up to 100 %.

7. Pharmaceutical formulation according to claim 1 to 6, in which the surfactant/ethanol ratio is in the range of from 73/27 to 77/23 and the concentration of compound of the formula (I) is in the range of from 5 to 25 mg/ml.

8. Pharmaceutical formulation according to one of claims 1 to 7 intended for dilution to form a perfusion solution.

9. Process for the preparation of a pharmaceutical formulation according to any one of claims 1 to 8, comprising the following steps:
- the surfactant is heated until it becomes liquid;
- the ethanol is added;
- the surfactant/ethanol mixture is cooled to ambient temperature;
- the compound of the formula (I) is added to the cooled mixture;
- the final mixture is sterilized.

10. Process according to claim 9, in which the mixture is sterilized by filtration.

11. Perfusion solution comprising the compound of the formula (I): 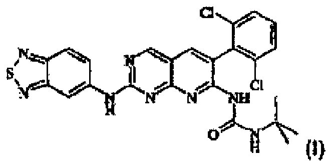 in the form of the base or in the form of a salt of a pharmaceutically acceptable acid,
obtained by dilution of 1 volume of the pharmaceutical solution according to claim 1 to 8 in 20 to 500 volumes of an isotonic solution.

12. Perfusion solution comprising the compound of the formula (I) : 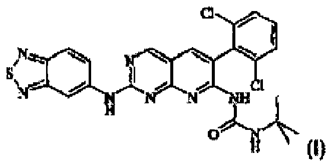 in the form of the base or in the form of a salt of a pharmaceutically acceptable acid, in a concentration in the range of from 0.01 to 1.2 mg/ml, the surfactant as defined in one of claims 1 to 7 in a concentration in the range of from 0.48 to 37 mg/ml and ethanol in a concentration in the range of from 0.35 to 35 mg/ml, diluted in an isotonic solution.

13. Perfusion solution according to claim 11 or 12 for administration to a human being.

14. Process for the preparation of a perfusion solution, consisting of dilution of 1 volume of the pharmaceutical solution according to claim 1 to 8 in 20 to 500 volumes of an isotonic solution.

15. Bottle containing the pharmaceutical solution as described in one of claims 1 to 8.

16. Drip bag containing the perfusion solution of claim 11 to 13.
